# EUROPEAN PATENT SPECIFICATION

(11) **EP 0 450 800 B1**
(45) Date of publication and mention of the grant of the patent: **26.06.1996**
(21) Application number: 91302397.4
(22) Date of filing: 20.03.1991
(51) Int. Cl.: A61K 47/48, A61K 7/28

(54) **Utilization and delivery of enzymes**
Verwendung und Verabreichung von Enzymen
Utilisation et délivrance d'enzymes

(30) Priority: 21.03.1990 GB 9006328; 05.10.1990 GB 9021671
(43) Date of publication of application: 09.10.1991
(73) Proprietor: UNILEVER PLC, London EC4P 4BQ (GB); UNILEVER N.V., NL-3013 AL Rotterdam (NL)
(72) Inventor: Beggs, Thomas Stewart, Elms Farm, Bedford MK41 0DN (GB); Davis, Paul James, Felmersham, Bedfordshire MK43 7EX (GB)
(74) Representative: Ford, Michael Frederick

(56) References cited:
- EP-A- 0 175 560
- EP-A- 0 315 364
- EP-A- 0 361 908
- WO-A-88/01178
- WO-A-89/11866
- FR-A- 2 187 294
- INFECTION AND IMMUNITY, vol. 27, no. 2, February 1980, pages 690-692; K. OKUDA et al.: "New type of antibody-enzyme conjugate which specifically kills Candida albicans"
- STN FILE SERVER & FILE MEDLINE & AN=81-192354, Derwent Publications Ltd, London, GB; R.B. JOHNSON, Jr. et al.: "Comparison of glucose oxidase and peroxidase as labels for antibody in enzyme-linked immunosorbent assay", & J. IMMUNOASSAY, (1980), 1(1), 27-37
- STN FILE SERVER & FILE CA & CHEMICAL ABSTRACTS, vol. 105, no. 17, abstract no. 145834w, Columbus, Ohio, US; J. PENE et al.: "In vitro cytolysis of myeloma tumor cells with glucose oxidase and lactoperoxidase antibody conjugates", & BIOCHEM. INT., 13(2), 233-43
- FILE WPIL & AN=88-043338, Derwent Publications Ltd, London, GB; & DD-A-249 977 (MEDIZIN AKAD. MAGDEB.)
- DD-A-249 977 (MEDIZIN AKAD. MAGDEB.)

## Description

### FIELD AND BACKGROUND OF THE INVENTION

This invention relates to the utilization of enzymes to perform a desired function, and to products for the purpose. An example of such a function is to attack species occurring in the oral microflora.

This invention entails use of two enzymes, one of which generates an intermediate product which is a substrate for the other enzyme. The latter enzyme converts the intermediate into an agent which is active against a target.

One possibility for the former enzyme is glucose oxidase. This catalyses the oxidation of glucose to gluconic acid by molecular oxygen, producing hydrogen peroxide in the process.

The other enzyme may then be a peroxidase, which functions to convert hydrogen peroxide into more potent oxidised species e.g. by reaction with halides such as iodide ion to produce hypoiodite or by reaction with thiocyanate to produce hypothiocyanate.

Hydrogen peroxide does display cytotoxicity but is rapidly decomposed in vivo. Oxidised species produced by peroxidase possess even greater toxicity but have an even shorter life in vivo.

### SUMMARY OF THE PRIOR ART

The use of enzymes to effect cell killing has already been proposed, and some of these proposals have sought to make use of a two-enzyme system.

Knowles et al, Journal of Clinical Investigation 52 1443 (1973), have described use of glucose oxidase chemically conjugated to antibodies capable of binding to target cells, thereby targeting the cell killing activity against those cells which it is desired to eliminate selectively. They provided peroxidase and halide in solution, and were able to demonstrate killing of bacterial cells.

Okuda et al, Infection and Immunity 27 690 (1980), have described the use of xanthine oxidase (which at least predominantly produces superoxide rather than peroxide) and lactoperoxidase chemically conjugated to antibody able to bind to target cells.

Over a period of 90 minutes, in vitro, they achieved a reduction of live Candida albicans cells, in vitro, which was between one and two orders of magnitude better than was achieved with lactoperoxidase in solution.

### SUMMARY OF THE INVENTION

In the present invention, one or more vehicles contain, in the same vehicle or distributed between a plurality of vehicles:
i) at least one of two enzymes which are an enzyme for generating an agent active against a target within the mouth, and a second enzyme for generating an intermediate which is a substrate for the first enzyme, and
ii) at least one antibody or antibody fragment able to bind to the site of the target at a surface within the mouth
   wherein each said enzyme is conjugated to a said antibody or antibody fragment or the product contains means to bind the enzyme to a said antibody or antibody fragment at the time of use whereby in use the enzymes are coupled to the target site in proximity to each other.

By means of this invention the two enzymes and the target are all held in proximity, so that the enzymes can co-operate to generate the active agent, e.g. a short lived oxidised halide species in proximity to the target site.

An aspect of the invention resides in a product comprising the above-mentioned vehicles. Another aspect is a method of delivering enzyme(s) by administration of the vehicles.

Both enzymes may be included in the vehicle or vehicles (not necessarily in the same vehicle). Alternatively one of the enzymes may be a material which is present in vivo in the general vicinity of the target site and in use becomes attached to the target site through a said antibody or antibody fragment. This would concentrate that enzyme into proximity with the other enzyme and the target site.

The enzymes which are used may be an oxidase which generates hydrogen peroxide plus a peroxidase which uses this as substrate, e.g. to form hypohalite. Superoxide and hydroxyl radicals can also be formed. A peroxidase may also use peroxide to convert thiocyanate to hypothiocyanate. Glucose oxidase is particularly envisaged. Another possibility is galactose oxidase, which could use as its substrate the galactose which occurs naturally in yoghurt.

Horseradish peroxidase is commercially available and could be used in conjunction with either of these oxidases. A further possibility is lactoperoxidase.

Various applications of the invention are envisaged, although the invention is not limited to these. One application which is particularly envisaged is to attack species of the supragingival oral microflora.

The oral microflora is a complex ecosystem which contains a wide variety of microbial species. One of these species may be selected as the target site. However, the effect of targeting to one species will be to attack both that species and other species which occur in close proximity to it. Thus, by delivering to one species which occurs in dental plaque, cytotoxic agents will be delivered to the plaque and will act against all the species which occur together in the plaque, including those responsible for plaque formation. Extra-cellular dextran produced by such organisms could itself be used as a target site, using an antibody or fragment with binding affinity for dextran.

One possible target site is Streptococcus mutans. This has been identified as an important contributor to dental plaque, and has been shown to be capable of inducing clinical caries lesions in germ free animals when established as a mono-infection. S. mutans has the ability to utilise dietary carbohydrate for the synthesis of an insoluble polysaccharide matrix, facilitating attachment to, and colonisation of, hard surfaces, as well as production of acids capable of the dissolution of enamel. These characteristics have been identified as important virulence determinants. Although other species and genera have also proved capable of both acid and plaque production, or even of caries initiation in the germ free animal, S. mutans is widely recognised as at least one significant cause of tooth decay because of the scale of its acid and polysaccharide production.

Other species which may be selected as the target species are S. sanguis, A. viscosus and A. naeslundii. These are all present in dental plaque as a substantial proportion of the species normally found in dental plaque. Because of frequent occurrence, these three may be preferred as target site.

Another application is to attack species of the subgingival microflora responsible for periodontal disease. The target species could well be Bacteroides gingivalis.

A possible cosmetic application is the reduction of stain on teeth. In this application, enzymes are used which produce a material with a bleaching function such as hypohalite ion. The target site is at the tooth surface. Binding to the target in accordance with this invention serves to locate the enzymes adjacent to the surface. In consequence a bleaching substance is produced in the vicinity of the tooth surface where staining may be present.

For any oral application (dental care) it would be necessary for the vehicle(s) in the product to be acceptable to be taken within the mouth, e.g. vehicle(s) suitable for topical application in the mouth.

### BRIEF DESCRIPTION OF THE DRAWINGS

The invention may be implemented in various ways. Some of these are illustrated in the accompanying drawings which are schematic diagrams of arrangements of targets and enzymes. G.Ox denotes glucose oxidase. HRP denotes horseradish peroxidase. PEI denotes polyethyleneimine.

In the drawings:
Figs. 1 to 4 each show different arrangements in which enzymes are attached to a target by whole antibodies;
Figs. 5 to 7 each show arrangements in which a complex formed using polyethyleneimine is attached to a target by whole antibodies; and
Fig. 8 shows enzymes attached to a target by antibody fragments.

### DETAILED DESCRIPTION OF EMBODIMENTS

As set forth above, a feature of this invention is that two co-operating enzymes become bound to a target site.

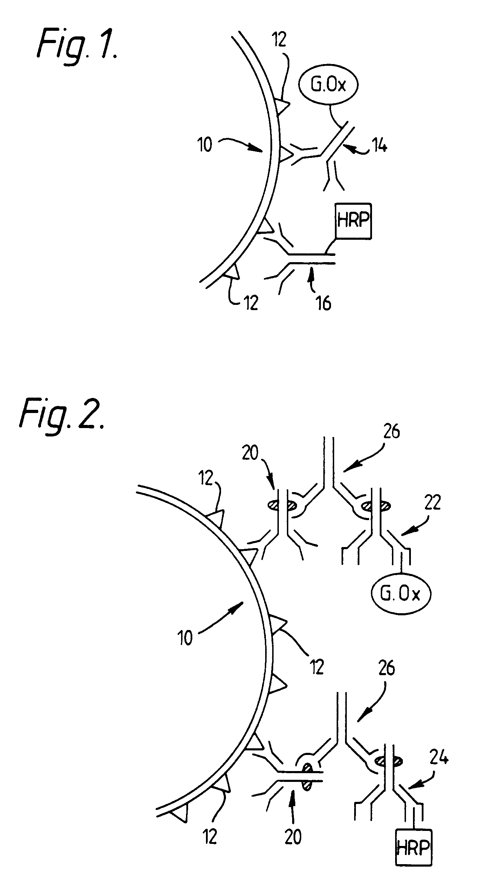

One possibility is that the enzymes are bound to the target site, but not linked to each other more directly, for instance if both enzymes are attached, e.g. conjugated through covalent bonds, to respective whole antibodies which are able to bind to the target site. This is illustrated in Figs.1 and 2.

In Fig. 1 numeral 10 denotes a target which is S. mutans with antigenic sites 12. Numeral 14 denotes anti-S. mutans antibody which has glucose oxidase (G.Ox) conjugated to it. Numeral 16 denotes anti-S. mutans antibody which has horseradish peroxidase (HRP) conjugated to it.

In Fig. 2 the need for chemical conjugation is avoided by the use of multiple antibodies. This is the subject of a co-pending application of even date herewith.

Numeral 20 denotes a first antibody which is rabbit anti-S. mutans. Numeral 22 denotes rabbit anti-glucose oxidase, bound to that enzyme. Numeral 24 denotes rabbit anti-horseradish peroxidase bound to horseradish peroxidase. Numeral 26 denotes goat anti-rabbit immunoglobulin which acts to bind antibodies 22 and 24 to anti-target antibodies 20.

Another possibility is that the product includes means to link the enzymes together, at least at the time of use. The linking means may be separate from but attached or attachable to an antibody which is able to bind to the target site.

It is preferred that the enzymes are linked together otherwise than by chemical conjugation to an antibody which binds directly to the target site (which may be a cell). Target-specific antibodies are likely to be less readily available than other antibodies or other materials which may be used for linking enzymes together. We may therefore prefer not to subject these target-specific antibodies to artificial chemical reactions used to form covalent bonds conjugating enzymes to antibodies.

Linking enzymes together otherwise than through the target site or target-specific antibodies can make it easier to control the distribution of enzymes and get the two kinds of enzyme in proximity to each other, so that the intermediate which is the product of one enzyme is generated in proximity to the other enzyme.

Hence, it can be advantageous that the linking means is one or more intermediaries to which the enzymes are attached or attachable and which are attached or attachable to antibody able to bind to the target site.

It is also preferred that the attachment of linking means to an antibody which binds to the target site is by means of antibody-antigen binding. This is beneficial in avoiding chemical conjugation to target specific antibody.

One preferred arrangement uses two more types of antibody besides the first mentioned antibody which binds to target sites. In this arrangement one enzyme (e.g. horseradish peroxidase) is attached, e.g. chemically conjugated through covalent bonding to a second antibody which can bind to the first antibody, while the other enzyme (e.g. glucose oxidase) is attached to third antibody able to bind to the second antibody. The linking means is then the combination of second and third antibodies.

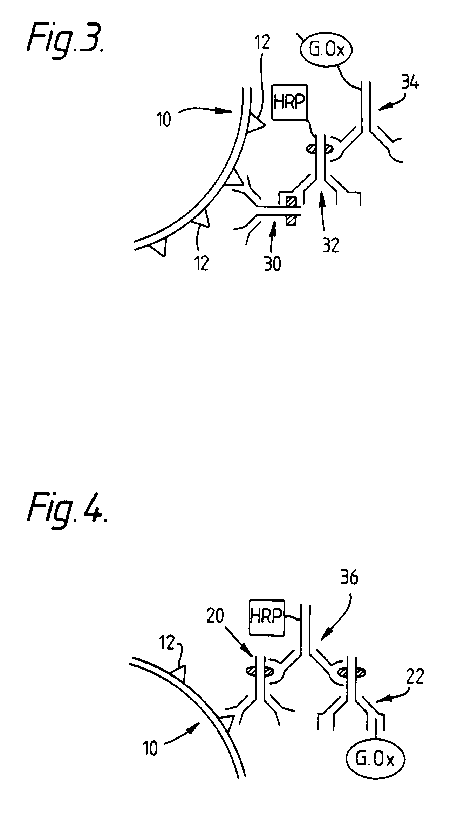

An example of this arrangement is shown in Fig. 3. Numeral 30 denotes bovine antibody to the S. mutans target 10. Numeral 32 denotes rabbit anti-bovine immunoglobulin which is conjugated to horseradish peroxidase and binds to the bovine antibody 30. Numeral 34 denotes sheep anti-rabbit immunoglobulin conjugated to glucose oxidase. This binds to antibody 32.

A variation is shown in Fig. 4 which is somewhat analogous to Fig. 2. Numerals 10, 12, 20 and 22 have the same significance as in Fig. 2, but numeral 36 denotes goat anti-rabbit immunoglobulin chemically conjugated to horseradish peroxidase.

Another possibility is for the two enzymes to be conjugated to a single intermediary which attaches to the first antibodies.

One possibility for such an intermediary is a synthetic polymer having chemical functionality to enable the attachment of enzymes by chemical reaction. A suitable polymer is polyethyleneimine which is a branched polymer with amino groups at the termini of the branches.

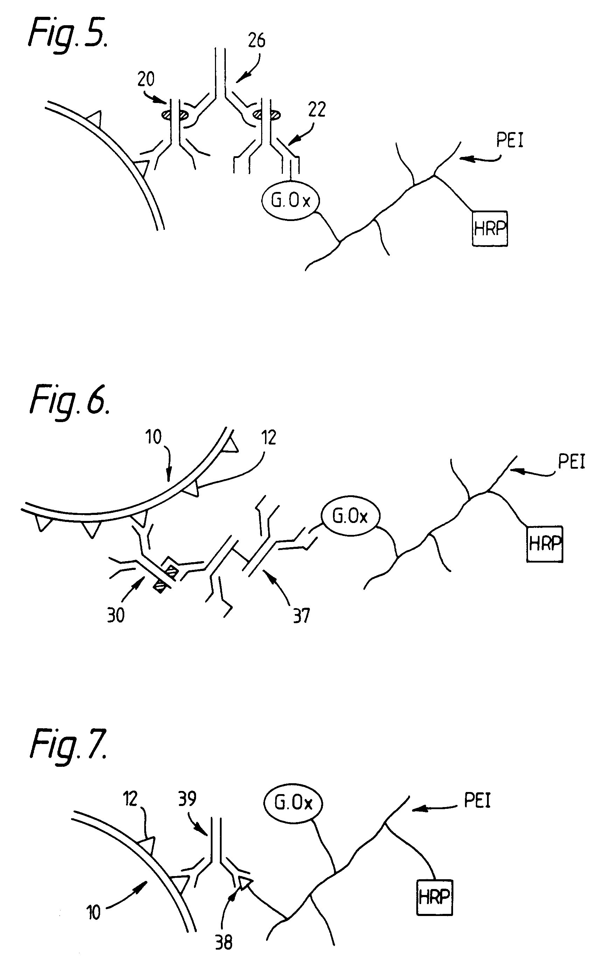

A complex of such polymer with both enzymes bound to it through covalent bonds could be attached to the target site in various ways. One possibility is for the product to include an antibody to one of the enzymes, and a further antibody able to form a bridge by binding both to this and to the antibody which binds to the target site. This is illustrated by Fig. 5 and is directly analogous to the arrangement of Fig. 2.

Another possibility is for the product to include a double antibody conjugate with two specificities which has the ability to bind to one of the enzymes and also to bind to the antibody which binds to the target. Double antibody conjugates are known per se. This arrangement is illustrated by Fig. 6. Numeral 30 denotes bovine antibody to the S. mutans target. Numeral 37 denotes a double antibody conjugate of anti-bovine and anti-glucose oxidase antibodies.

A further possibility is for the polyethyleneimine polymer to have antigenic sites characteristic of the target covalently bound to it. Antibody able to bind to the intended target would also bind to these antigenic sites on the polymer and in that way couple the polymer to the target site. This arrangement is illustrated by Fig. 7 where numeral 38 denotes an antigen attached to polyethyleneimine and numeral 39 denotes an anti-target antibody binding to the target 10 and to the antigen.

Glucose oxidase and horseradish peroxidase are both enzymes with pendant glycosyl chains. Such enzymes can be covalently bound to polyethyleneimine by first oxidising the enzymes in aqueous solution with periodate to generate aldehyde groups in the pendant glycosyl chains. These groups will then form Schiff bases with amino groups on the polyethyleneimine, at alkaline pH (e.g. pH 9.5) after which reduction with borohydride can be used to reduce any unreacted aldehyde groups and also increase stability by reduction of the Schiff bases.

These enzymes can also be conjugated to antibodies by the same technique.

Whole antibodies used in this invention may be polyclonal or monoclonal. Where an antibody of one specificity and an antibody of a different specificity are used together, it is possible that one antibody would be monoclonal while another antibody was polyclonal.

The invention can also be implemented using antibody fragments, notably utilising an antibody fragment to bind to the target site, together with one or more further fragments for binding enzymes to the first fragment.

The first antibody fragment which binds to a target site may be an Fv fragment of an antibody to the desired target. Such a fragment contains only the variable domains of light and heavy chains of an antibody. The fragment could possibly be an F(ab)₂ fragment which would provide two combining sites. It might alternatively be as little as a single variable domain of one chain of an antibody.

Techniques for efficient production of biologically active antibody fragments in E. coli were described by A Skevia and A. Pluckthun (1988), Science, 240, 1038; M. Better et al, (1988), Science 240, 1041; and E.S. Ward et al, (1989), Nature, 341, 544. The cloning and expression of genes encoding antibody fragments in E. coli is also described in published European application EP-A-368684 (Medical Research Council).

We prefer that a first antibody fragment has an additional peptide chain appended to it, and further fragments bind the enzymes to this peptide chain. Bacteria can be made to produce a variable domain with an extra peptide chain already attached to the C-terminus of the domain. This can be achieved by standard genetic techniques. For example, a gene encoding a variable domain can easily be lengthened by means of site directed mutagenesis techniques, using in vitro synthesised oligonucleotides which encode the peptide to be appended to the variable region. Site directed mutagenesis is now a widely used technique, and adequate protocols can be found in several published books, for example in Sambrook et al, (1989), Molecular Cloning, 2nd edition, Cold Spring Harbour Laboratory Press, New York. An attached extra peptide provides a very convenient "handle" for the attachment of the therapeutic agent.

Further antibody fragments which bind enzymes to the first fragment are preferably a second antibody fragment able to bind to an enzyme by antibody-antigen binding, and an F(ab)₂ fragment (which is bivalent) able to bind to the first and second antibody fragments, especially to antigenic peptides appended to the first and second antibody fragments.

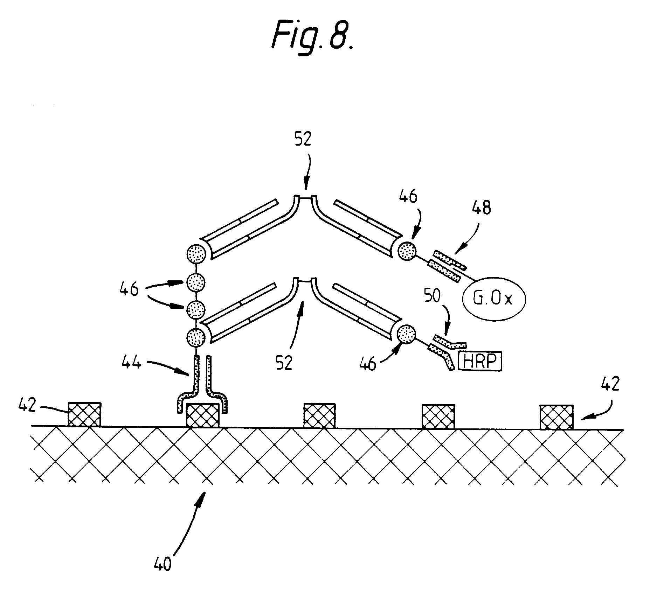

Fig. 8 illustrates a preferred arrangement in which antibody fragments are utilised.

For attaching to the target 40 which has antigenic sites 42 there is an Fv antibody fragment 44 with several repeats of a peptide 46 appended to the distal (C-terminal) end of one of the two chains in the Fv fragment 44.

Glucose oxidase (G.Ox) and horseradish peroxidase (HRP) are each bound by a respective Fv fragment 48,50 with specificity for the enzyme concerned, and with a single repeat of the same peptide 46 appended to one chain of the Fv fragment.

The peptides 46 appended to the anti-enzyme Fv fragments 48,50 become linked to peptides 46 on the anti-target Fv fragment by F(ab)₂ fragments 52 which bind specifically to these peptides. Since the F(ab)₂ fragments are divalent they can form a bridge attaching an anti-enzyme fragment, with attached enzyme, to the anti-target fragment 44.

If a plurality of polyclonal antibodies are used, it may be found desirable to distribute the enzymes and antibodies between more than one vehicle in the product , so that the full complexes of both enzymes and antibodies do not form until the time of use. We have found that during storage, large complexes with polyclonal antibodies are prone to suffer a reduction in their ability to bind to a target site.

Distribution of constituents of the complex between a plurality of vehicles may be unnecessary if monoclonal antibodies are employed. In general, monoclonal antibodies would form smaller complexes and during storage would be expected to retain their activity better than complexes formed with polyclonal antibodies. This is also true when antibody fragments are used. An advantage of antibody fragments is that the complexes which form by antigen - antibody binding are fairly small and more stable than complexes with whole antibodies.

When a product has the enzymes and one or more antibodies distributed between two vehicles, one of them could contain antibody able to bind to the target while the other vehicle could contain the enzymes and means to link them together, possibly as a preformed complex.

A product comprising a vehicle or vehicles containing enzymes and one or more antibodies or antibody fragments could take a number of forms. If the target site is in the mouth, possibilities include mouthwash, toothpaste and a lozenge which will dissolve in the mouth. These forms of product could be used even when a plurality of vehicles are needed. For instance the product could be a two-component mouthwash which the user mixes immediately before use.

It could be a toothpaste having two components stored in the toothpaste container in such a way that they are kept separate or at least do not mix but are dispensed together and mix in the mouth of the user. Such two-component toothpaste products are known per se.

Another possible form of product providing a plurality of vehicles would be a lozenge to be sucked in the mouth, with the various materials contained in separate regions of the lozenge.

Two vehicle forms could be used in combination as a way to provide a plurality of vehicles, e.g. toothpaste whose use is followed by a mouthwash or a lozenge.

The product may include some or all of the substrates for the enzymes, apart from the enzymatically generated intermediate, or it may rely on some or all enzyme substrates being present at the target site. Thus where glucose oxidase is used and the target site is in the mouth, the product could rely on dietary glucose as the enzyme substrate or it could itself incorporate glucose provided this was separate from the glucose oxidase.

### EXAMPLES

The invention is further explained by the following experimental Examples, demonstrating effectiveness of the system in vitro.

In the Examples "PBS" denotes phosphate buffered saline having pH 8 unless otherwise stated.

Dilutions are expressed in the form "1/n" signifying that a quantity of starting solution was mixed with diluent to give a solution in which the concentration was one n'th that of the starting solution.

### Example 1 : Part 1 - Materials employed

Horseradish peroxidase conjugated to rabbit anti-bovine immunoglobulin was a commercial product (Sigma).

Glucose oxidase conjugated to sheep anti-rabbit immunoglobulin was a commercial product (Serotec).

Bovine anti-S. mutans was prepared as follows: S. mutans was cultured overnight in Todd-Hewitt broth. The culture was killed with heat, mixed with sterile saline and fumed silica (Gasil) adjuvant to yield a solution containing 0.1g/ml Gasil and 10⁸ cells/ml. 2ml of this solution was injected intramuscularly into a calf. A similar injection was given three weeks later and after two more weeks antisera was extracted from whole blood. Rabbit anti-bovine immunoglobulin (used for comparisons) was also a commercial product (Sigma).

All reagent solutions, and PBS used for washing contained 0.15% v/v of the surfactant Tween 20 (polyoxyethylene (20) sorbitan monolaurate).

### Example 1 : Part 2 - Procedure

An experimental procedure was carried out in which S. mutans was exposed to bovine anti-S. mutans, then to materials to form a cell killing complex attached to the S. mutans through the bovine antibodies thereto.

Controls were carried out in which steps of the procedure were omitted or varied.

The procedure was a series of steps as follows:
1. 10ml aliquots of a culture of S. mutans in Todd Hewitt broth were transferred to sterile McCartney bottles. The bacteria were centrifuged into a pellet, washed 3 times with PBS at pH 8 and resuspended into 9ml PBS.
2. 1ml of bovine anti-S. mutans suspension was added. This suspension was a 1/10 dilution of whole serum in PBS at pH 8, sterilised by filtration. Alternatively, 1ml of PBS at pH 8 was added as control.
3. The suspension was incubated at room temperature for 1 hour, then the cells were centrifuged into a pellet and washed three times with PBS and resuspended in 9ml PBS as before.
4. 1ml of filter-sterilised horseradish peroxidase/rabbit anti-bovine conjugate, at 1/10 dilution in PBS at pH 8, was added giving a final dilution of 1/100. After 20 minutes incubation at room temperature the cells were centrifuged into a pellet, washed 3 times with PBS at pH 8 and resuspended in 9ml PBS as before.
   In a control, 1ml of PBS was added in place of the conjugate. In comparative experiments the conjugate was replaced with rabbit anti-bovine immunoglobulin.
5. 1ml of filter sterilised glucose oxidase/sheep anti-rabbit conjugate, at 1/10 dilution in PBS at pH 8 was now added, giving a final dilution of 1/100 after addition. Again, the suspension was incubated at room temperature for 20 minutes, then the cells were centrifuged into a pellet and washed 3 times with PBS.
6. The cells were resuspended in 10ml of filter sterilised PBS at pH 6.5 containing 15µg/ml potassium iodide and 5% w/v D-glucose.
7. The resulting suspension was incubated at 37°C for 24 hours. 0.5ml samples were taken immediately on mixing, and at intervals. The bacteria in each sample were separated by centrifugation into a pellet which was washed three times with PBS. The viable cells in each sample were assayed by the method of Miles, Misra and Irwin J. Hygiene 38, 732-749, (1938). The combinations of materials added, and the counts of viable cells are set out in the following Table.
8. Samples of the suspension produced in step 6 were assayed colorimetrically for the simultaneous presence of both enzymes bound to the cells. The cells were centrifuged at 4000rpm for 5 minutes and then washed 3 times by resuspending the pellet in 3ml of PBS, and centrifuging again. After the last centrifugation the cells were resuspended in 0.5ml of PBS containing 100mM glucose and 1µg/ml of tetramethyl benzidine. This system develops colour only where glucose oxidase and peroxidase are present together. Colour was allowed to develop for 5 minutes, then stopped by addition of 50µl of 0.2M HCl. Optical densities were determined and are included in Table 1.

**TABLE 1**

| Step 2 | Step 4 | Step 5 | Optical Density in colorimetric assay | NUMBERS OF SURVIVING CELLS Duration of Incubation (hours) | | | | | |
|---|---|---|---|---|---|---|---|---|---|
| | | | | 0 | 1 | 2 | 4 | 8 | 24 |
| Ab | PBS | PBS | 0 | 5.4x10⁷ | 3.2x10⁷ | 2x10⁷ | 2x10⁷ | 2x10⁷ | 6x10⁶ |
| Ab | HRP conj | G.ox conj | 1.079 | 9.8x10⁶ | 3.6x10⁵ | 2x10¹ | 6x10¹ | 2.2x10² | 0 |
| PBS | HRP conj | G.ox conj | 0.004 | 2.4x10⁷ | 1.2x10⁷ | 1.2x10⁶ | 5.8x10⁵ | 3x10² | 1x10² |
| Ab | anti-bov | G.ox conj | 0.002 | 8.8x10⁶ | 1.8x10⁵ | 2x10³ | 4x10² | 2.8x10² | 4x10¹ |
| PBS | anti-bov | G.ox conj | 0.015 | 2.3x10⁷ | 1.4x10⁷ | 2.8x10⁶ | 1.6x10² | 8x10¹ | 8x10² |
| Ab | HRP conj | PBS | 0.072 | 3.2x10⁷ | 2.2x10⁷ | 1.04x10⁷ | 2x10⁶ | 1.2x10⁵ | 1.4x10⁴ |
| Ab | PBS | G.ox conj | 0.002 | 2.2x10⁷ | 2.6x10⁷ | 1.6x10⁷ | 1.2x10⁷ | 2.4x10⁴ | 2x10² |

As can be seen from the Table, the cells generally survive in the presence of antibodies to them (experiment A). When glucose oxidase conjugate is present, but unable to bind to the S. mutans cells (experiment G) it displays a cell killing effect. It displays a greater cell killing effect if bound to the target S. mutans cells (experiment D).

Surprisingly, horseradish peroxidase conjugate produced some cell killing without glucose oxidase (experiment F). In experiment C both conjugates were present and able to complex together but unable to attach to the target S. mutans cells: cell killing activity was greater than with unbound glucose oxidase conjugate or with peroxidase conjugate (experiments G and F).

However, much greater cell killing is achieved by experiment B which contains all the components to enable both conjugates to attach together and also attach to the target cells. The number of surviving cells dropped dramatically within two hours, and eventually fell to zero.

### Example 2

The procedure of Example 1 was repeated, using two different dilutions of the glucose oxidase/sheep anti-rabbit conjugate. In some experiments the conjugate used was 1/10 dilution in PBS at pH 8, so that after addition the final dilution of the conjugate was 1/100. In other experiments the conjugate was used at 1/160 dilution, so that after addition the final dilution was 1/1600.

The combinations of materials added, the counts of viable cells, and optical densities from the colorimetric assay are set out in the following Table.

**TABLE 2**

| Step 2 | Step 4 | Step 5 | Optical Density in colorimetric assay | NUMBERS OF SURVIVING CELLS PER ML Duration of Incubation (hours) | | | | | |
|---|---|---|---|---|---|---|---|---|---|
| | | | | 0 | 1 | 2 | 4 | 8 | 24 |
| Ab | PBS | PBS | 0.03 | 4x10⁶ | 1.32x10⁶ | 1.24x10⁶ | 5.6x10⁶ | 1x10⁶ | 2x10⁵ |
| Ab | HRP conj | 1/10 G.ox | 12.7* | 8.4x10⁵ | 4x10¹ | 0 | 1.1x10³ | 0 | 6x10¹ |
| PBS | HRP conj | 1/10 G.ox | 0.02 | 5.6x10⁶ | 2.8x10⁶ | 3x10⁶ | 2.8x10⁶ | 2.6x10⁵ | 0 |
| Ab | HRP conj | 1/160 G.ox | 7.7* | 7.6x10⁵ | 5.2x10³ | 0 | 2x10¹ | 0 | 1.2x10² |
| PBS | HRP conj | 1/160 G.ox | 0.07 | 6x10⁶ | 4.2x10⁶ | 2.2x10⁶ | 1.2x10⁶ | 2.2x10⁶ | 7x10⁵ |
| Ab | PBS | 1/160 G.ox | 0.03 | 2x10⁶ | 1.08x10⁶ | 1.2x10⁶ | 5.2x10⁵ | 4.8x10⁵ | 1.4x10⁵ |
| PBS | PBS | 1/160 G.ox | 0.02 | 2.8x10⁶ | 2.4x10⁶ | 6.2x10⁶ | 4x10⁶ | 2x10⁶ | 5.4x10⁵ |

| | | | | | | | | | |
|---|---|---|---|---|---|---|---|---|---|
| * Calculated values derived from actual values measured at higher dilution. | | | | | | | | | |

It can be seen from Table 2 that cells survive in the presence of antibodies to them (experiment A) and that the dilute glucose oxidase conjugate displays little cell killing activity in the absence of peroxidase (experiments F and G).

Unattached complex of peroxidase conjugate with the dilute glucose oxidase conjugate (experiment E) displays markedly less cell killing than a complex using the more concentrated glucose oxidase conjugate (experiment C). Much faster cell killing was observed with complex bound to the S. mutans cells (experiments B and D). It is particularly notable that bound complex with the dilute glucose oxidase conjugate (experiment D) was considerably more effective than unbound complex with the more concentrated conjugate (experiment C).

### Example 3

This Example demonstrates the killing of cells using glucose oxidase and peroxidase, both targeted to the cells but not otherwise linked together. The cells in this Example were human tumour cells (cultured LoVo cells) but the Example is also a model for the killing of other cells.

LoVo cells were detached from a tissue culture and suspended in RPMI 1640 tissue culture medium. Aliquots of the suspension were pipetted into wells of a tissue culture plate which was incubated for 4 days at 37°C for LoVo cells to attach to the plate. The culture medium was tipped off the culture plate and an aliquot of mouse anti-LoVo antibody suspension was added to each well. This antibody was a monoclonal antibody, designated AUA-1 (ex Unipath Ltd). Each aliquot was 200µl of a 1/1000 dilution of AUA-1 antibody suspension.

The plate was incubated for 1 hour at 37°C, then the antibody suspension was tipped off. Each well next received a 200µl aliquot of a solution containing the following:

| | |
|---|---|
| glucose oxidase (G.Ox) | 10µg/ml |
| horseradish peroxidase (HRP) | 10µg/ml |
| mouse anti-G.Ox antibody | 1250ng/ml |
| mouse anti-HRP antibody | 250ng/ml |
| rabbit anti-mouse antibody | 50µg/ml |

It would be expected that two types of complex would form by antibody-antigen binding, viz:
a) LoVo:Mouse anti-LoVo:Rabbit anti-mouse:Mouse anti-G.Ox:G.Ox
b) LoVo:Mouse anti-LoVo:Rabbit anti-mouse:Mouse anti-HRP:HRP

Some wells were used as controls, and received an aliquot of a solution containing only the enzymes.

Every well next received a 50µl portion of a catalase solution. Various catalase concentrations were used. The catalase functioned to destroy hydrogen peroxide.

The plate was incubated for 1 hour at 37°C after which the extent to which the LoVo cells remained viable was determined. To do this 100µl of solution was removed from each well and replaced by 50µl of a 1mg/ml solution of: (3-(4,5-dimethylthiazole-2-yl) 2,5-diphenyl) tetrazolium bromide (MTT) (Sigma Chemical Co). Living cells generated blue crystals of formazan. These blue crystals were dissolved by addition of 100µl of 0.04 N HCl in isopropanol to each well. The optical density was read on an ELISA plate reader at 570 nm.

The results were:

| Catalase concentration (µg/ml⁾ | Absorbance at 570nm | |
|---|---|---|
| | Enzyme solution | Enzymes and antibodies |
| 0 | L | L |
| 2.5 | H | L |
| 5 | H | L |
| 10 | H | L |
| 20 | H | L |
| 40 | H | H |
| 80 | H | H |
| L = 0.2 within experimental error H = 1.1 within experimental error | | |

Thus, without catalase present, even the free enzymes killed the LoVo cells. At 40µg/ml or more of catalase, its scavenging of H₂O₂ suppressed all cell killing. Lower concentrations of catalase suppressed cell killing by free enzymes but not when the antibodies were present. This demonstrates that the enzymes were able to kill cells and that the antibodies were making the enzymes more effective by attaching them to the target cells.

### Example 4

Like Example 3, this example demonstrates the killing of cells using glucose oxidase and a peroxidase, both targeted to the cells. The cells in this example were S. sanguis which is a species that occurs in the mouth. The antibodies used in this example were made by standard techniques.

S. sanguis cells were centrifuged out of a culture broth, washed three times by resuspending in PBS and centrifuging, then resuspended in PBS. Aliquots were placed in each of a number of tubes, centrifuged, and resuspended in a filter sterilized suspension (in PBS) of mouse polyclonal anti-S. sanguis antibodies (obtained from ascites fluid). The suspensions were incubated at room temperature for 30 minutes to allow the antibodies to bind to the cells. The cells were again centrifuged and washed with PBS.

Some tubes, used as a comparison, received PBS in place of the mouse anti-S. sanguis suspension.

Next the cells were resuspended in 1ml of PBS containing

| | |
|---|---|
| glucose oxidase (G.Ox) | 100µg/ml |
| horseradish peroxidase (HRP) | 100µg/ml |
| mouse anti-G.Ox monoclonal antibody | 1.25µg/ml |
| mouse anti-HRP monoclonal antibody | 1.25µg/ml |
| rabbit anti-mouse polyclonal antibody | 50µg/ml |

(The rabbit anti-mouse antibody was added to a solution already containing the other four materials).

In some tubes PBS alone was substituted for the above solution containing enzymes and antibodies. In further tubes a solution containing enzymes and antibodies was used but rabbit anti-mouse antibody was omitted.

All of the suspensions were incubated at room temperature for 30 minutes to allow the enzymes to become bound to the cells. Then the cells were centrifuged out, washed with PBS and resuspended in 1ml of PBS containing 15µg/ml potassium iodide and 5%w/v glucose.

These suspensions were incubated for 24 hours at room temperature. Sample portions were removed from each suspension at the beginning of this incubation period and after periods of 1,2,3,4 and 24 hours. The viable cells in each sample were assayed in the same way as in Example 1 and the results are set out in the following Table 3.

**TABLE 3**

| | Anti-target | Enzymes and antibodies | SURVIVING CELLS PER ML Duration of Incubation (hours) | | | | | |
|---|---|---|---|---|---|---|---|---|
| | | | 0 | 1 | 2 | 3 | 4 | 24 |
| A | Mouse anti-S. sanguis | None | 5x10⁷ | 1.4x10⁷ | 4.2x10⁷ | 2.2x10⁶ | 4x10⁷ | 4.6x10⁵ |
| B | Mouse anti-S. sanguis | All | 4.4x10⁷ | 4x10⁶ | 2.4x10⁷ | 4.6x10⁵ | 1.6x10⁵ | 1 |
| C | Mouse anti-S. sanguis | Rabbit anti-mouse omitted | 5x10⁷ | 6x10⁶ | 3.6x10⁷ | 7.4x10⁶ | 2.6x10⁷ | 2.4x10⁴ |
| D | None | All | 5.2x10⁷ | 6.4x10⁶ | 2.6x10⁷ | 2.8x10⁶ | 8x10⁶ | 4.6x10⁴ |

Sample B, with both enzymes attached to the target cells gave much more effective cell killing than in the other samples.

### Example 5

This example provides an in vitro model of the use of two targeted enzymes to bleach tooth stain. A nitrocellulose membrane was used as the model surface.

The membrane was incubated with normal mouse serum for one hour. Such serum contains mouse immunoglobulin. Consequently mouse antibodies became attached to the membrane. The membrane was then incubated with a 3% solution of bovine serum albumin to block any remaining protein binding sites on the membrane. Samples of membrane treated as above were used as control and test samples (4 of each).

Next the test samples of the membrane (but not the control samples) were incubated with a solution containing:

| | |
|---|---|
| glucose oxidase (G.Ox) | 100µg/ml |
| horseradish peroxidase (HRP) | 100µg/ml |
| mouse anti-G.Ox monoclonal antibody | 1.25µg/ml |
| mouse anti-HRP monoclonal antibody | 1.25µg/ml |
| rabbit anti-mouse polyclonal antibody | 50µl/ml |

similar to that used in the previous Example.

This led to formation of complexes as illustrated in Fig. 2, with nitrocellulose membrane providing the target.

The test samples and the control samples were next stained with tea. An infusion of leaf tea (2% by weight) in boiling water was left to cool to room temperature and incubated with the test and control samples for 5 minutes, after which the samples were rinsed in PBS for 1 hour to remove unbound colour.

The samples were incubated in a PBS solution of pH 7.2 containing 5.4% by weight glucose overnight (18 hours). It was expected that this would provide substrate for the bound enzymes, leading to production of reactive species in the vicinity of the tea stain.

The intensity of the stain of the samples was assessed before and after incubation with the glucose solution, using a Minolta CR 200 chromometer to assess reflectance. The result is expressed on a numerical scale.

The observed increases in reflectance, and the standard deviations were:

| | |
|---|---|
| Test samples (with enzymes) | 1.17 ± 0.042 |
| Control samples | 0.15 ± 0.31 |

This shows a statistically significant (p < 0.05) increase in brightness of the test samples, compared to the control samples.

## Claims

1. A product comprising one or more vehicles containing, in the same vehicle or distributed between a plurality of vehicles:
i) at least one of two enzymes which are an enzyme for generating an agent active against a target within the mouth and a second enzyme for generating an intermediate which is a substrate for the first enzyme, and
ii) at least one antibody or antibody fragment able to bind to the site of the target at a surface within the mouth,
wherein each said enzyme is attached to a said antibody or antibody fragment or the product contains means to bind the enzyme to said antibody or antibody fragment at the time of use whereby in use the enzymes are coupled to the target site in proximity to each other.

2. A product according to claim 1 wherein the first enzyme is an oxidase which generates hydrogen peroxide and the second enzyme is a peroxidase.

3. A product according to claim 1 or claim 2 including both enzymes.

4. A product according to any one of claims 1 to 3 wherein each enzyme is chemically conjugated to an antibody.

5. A product according to any one of claims 1 to 3 wherein at least one of the enzymes is chemically conjugated to an antibody which attaches by antibody-antigen binding to an antibody which is able to bind to the target site.

6. A product according to any one of claims 1 to 5 including means to link the enzymes to each other, the linking means being separate from, but attached or attachable to an antibody or antibody fragment which is able to bind to the target site.

7. A product according to claim 6 wherein attachment of the linking means to the antibody or antibody fragment able to bind to the target site is through antibody-antigen binding.

8. A product according to claim 7 wherein one enzyme is attached or attachable to a second antibody which binds to the antibody which is able to bind to the target site and the other enzyme is attached or attachable to a third antibody which binds to the second antibody.

9. A product according to claim 8 wherein at least one enzyme is chemically conjugated to its respective antibody.

10. A product according to claim 6 wherein both enzymes are chemically conjugated to a carrier material which provides the said linking means.

11. A product according to claim 10 wherein the carrier material is polyethyleneimine.

12. A product according to any one of the preceding claims wherein the target site is an oral bacterial species.

13. A product according to any one of the preceding claims, wherein the or every vehicle is acceptable to be taken within the mouth.

14. A product according to claim 13 which is, or in which at least one vehicle is provided by, a toothpaste, a mouthwash or a lozenge.

15. A cosmetic method of reducing tooth stain which comprises administering a product according to any one of the preceding claims.

16. A cosmetic method of reducing tooth stain which comprises administering
i) at least one of two enzymes which are an enzyme for generating a bleaching agent active against tooth stain, and a second enzyme for generating an intermediate which is a substrate for the first enzyme, and
ii) at least one antibody or antibody fragment able to bind to the site of the target stain at tooth surfaces,
wherein each said enzyme is attached to a said antibody or the product contains means to bind the enzyme to said antibody or antibody fragment at the time of use whereby in use the enzymes are coupled to the target site in proximity to each other.

17. Use of at least one vehicle, at least one antibody or fragment thereof and at least one enzyme, all as specified in claim 1 to prepare a product for attacking a target at a surface within the mouth.

## Patentansprüche

1. Ein Produkt, einschließend einen oder mehrere Träger, enthaltend in dem gleichen Träger oder verteilt zwischen einer Vielzahl von Trägern:
(i) Zumindest eines von zwei Enzymen, die ein Enzym zur Erzeugung eines aktiven Mittels gegen ein Target innerhalb des Munds sind, und ein zweites Enzym zur Erzeugung eines Zwischenprodukts, welches ein Substrat für das erste Enzym ist, und
(ii) zumindest einen Antikörper oder ein Antikörperfragment, fähig, sich an den Ort des Targets an einer Oberfläche innerhalb des Munds zu binden,
worin jedes erwähnte Enzym an einen vorerwähnten Antikörper oder ein Antikörperfragment gebunden ist oder das Produkt Mittel zum Binden des Enzyms an einen erwähnten Antikörper oder an das Antikörperfragment zum Zeitpunkt der Verwendung enthält, wodurch die Enzyme bei der Verwendung mit der Targetstelle in geringem Abstand zueinander verbunden sind.

2. Ein Produkt nach Anspruch 1, worin das erste Enzym eine Oxidase ist, welche Wasserstoffperoxid erzeugt und das zweite Enzym eine Peroxidase ist.

3. Ein Produkt nach Anspruch 1 oder Anspruch 2, enthaltend beide Enzyme.

4. Ein Produkt nach einem der Ansprüche 1 bis 3, worin jedes Enzym chemisch an einen Antikörper gebunden ist.

5. Ein Produkt nach einem der Ansprüche 1 bis 3, worin zumindest eines der Enzyme chemisch an einen Antikörper gebunden ist, welcher durch Antikörper-Antigen-Bindung an einen Antikörper angeschlossen ist, der zur Bindung an die Targetstelle fähig ist.

6. Ein Produkt nach einem der Ansprüche 1 bis 5, einschließend Mittel zur Verbindung der Enzyme miteinander, wobei die Bindungsmittel getrennt von, jedoch verknüpft oder verknüpfbar an einem Antikörper oder an ein Antikörperfragment sind, welches zur Verbindung mit der Targetstelle fähig ist.

7. Ein Produkt nach Anspruch 6, worin die Verknüpfung des Bindungsmittels an den Antikörper oder an das Antikörperfragment, fähig, sich an die Targetstelle zu binden, durch Antikörper-Antigen-Bindung erfolgt.

8. Ein Produkt nach Anspruch 7, worin ein Enzym verknüpft oder verknüpfbar an einen zweiten Antikörper ist, der zu dem Antikörper bindet, der fähig ist, sich mit der Targetstelle zu verbinden, und das andere Enzym mit einem dritten Antikörper verknüpft oder verknüpfbar ist, welcher sich mit dem zweiten Antikörper verbindet.

9. Ein Produkt nach Anspruch 8, worin zumindest ein Enzym chemisch an seinen entsprechenden Antikörper gebunden ist.

10. Ein Produkt nach Anspruch 6, worin beide Enzyme chemisch an ein Trägermaterial gebunden sind, welches die erwähnten Bindungsmittel liefert.

11. Ein Produkt nach Anspruch 10, worin das Trägermaterial Polyethylenimin ist.

12. Ein Produkt nach einem der vorstehenden Ansprüche, worin die Targetstelle eine orale bakterielle Spezies ist.

13. Ein Produkt nach einem der vorstehenden Ansprüche, worin der oder jeder Träger verträglich für die Aufnahme in den Mund ist.

14. Ein Produkt nach Anspruch 13, welches ist, oder in welchem zumindest ein Träger vorgesehen ist, eine Zahnpasta, ein Mundwasser oder eine Rautenpastille.

15. Ein kosmetisches Verfahren zur Reduzierung von Zahnverfärbung, welches das Verabreichen eines Produkts gemäß einem der vorstehenden Ansprüche umfaßt.

16. Ein kosmetisches Verfahren zur Reduzierung von Zahnverfärbung, welches umfaßt das Verabreichen
(i) von zumindest einem oder zwei Enzymen, die ein Enzym zur Erzeugung eines aktiven Bleichmittels gegen Zahnverfärbung sind, und eines zweiten Enzyms zur Erzeugung eines Zwischenprodukts, welches ein Substrat für das erste Enzym ist, und
(ii) von zumindest einem Antikörper oder einem Antikörperfragment, fähig, sich zu binden an die Stelle der Targetflecken an den Zahnoberflächen,
worin jedes erwähnte Enzym gebunden ist an den erwähnten Antikörper oder das Produkt Mittel enthält, das Enzym an den erwähnten Antikörper oder an das Antikörperfragment zum Zeitpunkt der Verwendung zu binden, wobei im Gebrauch die Enzyme mit der Targetstelle in der Nähe zueinander verbunden sind.

17. Die Verwendung von zumindest einem Träger, zumindest einem Antikörper oder einem Fragment desselben und von zumindest einem Enzym, alle wie sie in Anspruch 1 spezifiziert sind, zur Herstellung eines Produkts zum Angreifen eines Targets an einer Oberfläche innerhalb des Munds.

## Revendications

1. Produit comprenant un ou plusieurs véhicules contenant, dans le même véhicule ou répartis entre plusieurs véhicules
i) au moins une de deux enzymes qui sont une enzyme pour engendrer un agent actif contre une cible à l'intérieur de la bouche, et une enzyme pour engendrer un intermédiaire qui est un substrat pour la première enzyme, et
ii) au moins un anticorps ou fragment d'anticorps capable de se lier au site de la cible à une surface à l'intérieur de la bouche.
dans lequel chaque dite enzyme est fixée audit anticorps ou fragment d'anticorps ou bien le produit contient un moyen pour lier l'enzyme audit anticorps ou fragment d'anticorps au moment de l'utilisation, ainsi à l'utilisation les enzymes sont couplées au site cible à proximité l'une de l'autre.

2. Produit selon la revendication 1, dans lequel la première enzyme est une oxydase qui engendre du peroxyde d'hydrogène et la seconde enzyme est une peroxydase.

3. Produit selon la revendication 1 ou la revendication 2, comprenant les deux enzymes.

4. Produit selon l'une quelconque des revendications 1 à 3, dans lequel chaque enzyme est chimiquement conjuguée à un anticorps.

5. Produit selon l'une quelconque des revendications 1 à 3, dans lequel au moins une des enzymes est chimiquement conjuguée à un anticorps qui se fixe par une liaison anticorps-antigène à un anticorps qui est capable de se lier au site cible.

6. Produit selon l'une quelconque des revendications 1 à 5 comprenant un moyen de lier les enzymes entre elles, le moyen de liaison étant séparé de, mais fixé ou fixable à un anticorps ou fragment d'anticorps qui est capable de se lier au site cible.

7. Produit selon la revendication 6, dans lequel la fixation du moyen de liaison à l'anticorps ou au fragment d'anticorps capable de se lier au site cible est réalisée par l'intermédiaire d'une liaison anticorps-antigène.

8. Produit selon la revendication 7, dans lequel une enzyme est fixée ou fixable à un deuxième anticorps qui se lie à l'anticorps qui est capable de se lier au site cible et l'autre enzyme est fixé ou fixable à un troisième anticorps qui se lie au deuxième anticorps.

9. Produit selon la revendication 8, dans lequel au moins une enzyme est chimiquement conjuguée à son anticorps respectif.

10. Produit selon la revendication 6, dans lequel les deux enzymes sont chimiquement conjuguées à un matériau porteur qui fournit ledit moyen de liaison.

11. Produit selon la revendication 10, dans lequel le matériau porteur est le polyéthylèneimine.

12. Produit selon l'une quelconque des revendications précédentes, dans lequel le site cible est une espèce bactérienne buccale.

13. Produit selon l'une quelconque des revendications précédentes, dans lequel le ou chaque véhicule est acceptable pour être pris à l'intérieur de la bouche.

14. Produit selon la revendication 13 qui est, ou dans lequel au moins un des véhicules est fourni par, une pâte dentifrice, un bain de bouche ou une pastille.

15. Procédé cosmétique de réduction des taches sur les dents qui comprend l'administration d'un produit selon l'une quelconque des revendications précédentes.

16. Procédé cosmétique de réduction des taches sur les dents qui comprend l'administration
i) d'au moins une de deux enzymes qui sont une enzyme pour engendrer un agent de blanchiment actif contre les taches sur les dents, et une seconde enzyme pour engendrer un intermédiaire qui est un substrat pour la première enzyme, et
ii) au moins un anticorps ou un fragment d'anticorps capable de se lier au site cible de la tache à la surface de la dent,
dans lequel ladite enzyme est fixée audit anticorps, ou bien le produit contient un moyen pour lier l'enzyme audit anticorps ou fragment d'anticorps au moment de l'utilisation ainsi à l'utilisation les enzymes sont couplées au site cible à proximité l'une de l'autre.

17. Utilisation d'au moins un véhicule, au moins un anticorps ou fragment de celui-ci et au moins une enzyme, tous comme spécifiés dans la revendication 1 afin de préparer un produit pour attaquer une cible à une surface à l'intérieur de la bouche.
